# EUROPEAN PATENT APPLICATION

(11) **EP 4 826 384 A2**
(43) Date of publication of application: **21.10.2026**
(21) Application number: 26195169.3
(22) Date of filing: 19.05.2021
(51) Int. Cl.: G01N 33/543

(54) **METHOD OF DETECTING ANALYTES IN A SAMPLE**

(30) Priority: 29.05.2020 IN 202011022585
(62) Document number(s) of the earlier application(s) in accordance with Art. 76 EPC: 21812454.3 / 4 158 052
(71) Applicant: Siemens Healthcare Diagnostics, Inc., Tarrytown, NY 10591 (US)
(72) Inventor: KHANDIGE, Divya, 560032 Karnataka (IN); MITRA, Nivedita, 560066 Karnataka (IN); V M, Ramya, 560019 Karnataka (IN)
(74) Representative: Platen, Denise

(57) **Abstract**

A method and a kit for detecting one or more analytes in a sample is disclosed. In one aspect, the method includes introducing the sample to a surface bound to at least one portion of a first antibody to form a first antibody-analyte complex. The method further includes incubating the first antibody-analyte complex with a set of second antibodies to form a first antibody-analyte-second antibody complex, wherein one second antibody is conjugated with a nucleic acid fragment comprising an exposed 3' hydroxyl group and another second antibody is conjugated with an exposed 5' phosphate group. Additionally, the method includes ligating the nucleic acid fragment comprising the exposed 3' hydroxyl group and the nucleic acid fragment comprising the exposed 5' phosphate group. Furthermore, the method includes separating the ligated nucleic acid fragments from the first antibody-analyte-second antibody complex.

## Description

### FIELD OF TECHNOLOGY

The present invention relates to a method of detecting analytes in a sample. In particular, the invention relates to quantifying the amount of analytes that may be present in a given sample.

### BACKGROUND

Effective and quick detection of clinically relevant analytes is the need of the hour, Early diagnosis of diseases requires the diagnostic methods to be sensitive to minute quantities of analytes in physiological samples such as whole blood, urine, sputum, etc. Current methods provide for early diagnosis of diseases. However, such methods involve multiple steps in the workflow, thereby increasing the complexity of the solution. Currently available methods such as antibody detection by agglutination-PCR (ADAP) involve binding of antibodies to synthetic antigen-DNA conjugates, enabling ligation of the DNA strands and subsequent quantification by quantitative PCR (qPCR). However, the specificity of this method may be low.

Therefore, there exists a need for a method that enables detection of analytes in a sample in minute quantities, that is effective and simple.

The object of the invention is therefore to provide a method that enables detection of analytes such as proteins in a given sample that is quick, effective and has a high sensitivity.

### SUMMARY

The invention achieves the object by a method of detection of one or more analytes in a sample. The method includes introducing the sample to a surface bound to one or more first antibodies, wherein the surface is bound to at least one portion of each first antibody. The first antibody may be chosen such that the affinity of the first antibody towards the one or more analyte in the sample is high. The first antibody may bind to the analyte in the sample to form a first antibody-analyte complex. The method further includes incubating the first antibody-analyte complex with at least one portion of a second antibody. The second antibody may be conjugated with a nucleic acid fragment comprising an exposed 3' hydroxyl group, while another second antibody may be conjugated with a nucleic acid fragment comprising an exposed 5' phosphate group. In an embodiment, multiple second antibodies bind with the first antibody-analyte complex to form first antibody-analyte-second antibody complex. The method enables the first antibody-analyte-second antibody complex which in turn helps in bringing the second antibody tethered nucleic acids in close proximity.

The method further includes ligating the nucleic acid fragment comprising the exposed 3' hydroxyl group with the nucleic acid fragment comprising the exposed 5' phosphate group. In an embodiment, the nucleic acid fragment comprising the 3' hydroxyl group may be in proximity to the nucleic acid fragment comprising the 5' phosphate group. The method further includes separating the ligated nucleic acid fragments from the first antibody-analyte-second antibody complex. The separation may be achieved, for example, via alkaline hydrolysis. Alternatively, the ligated nucleic acid fragments may be separated during an amplification process of the nucleic acid fragments. The separation is achieved such that the nucleic acid fragments conjugated to the second antibody is dissociated from the second antibody. The method further includes amplifying the ligated nucleic acid fragments and detecting the one or more analytes present in sample based on the amplified nucleic acid fragments. The nucleic acid hence acts as a surrogate for the target analyte.

The present invention is advantageous over ADAP in that the present invention uses at least one portion of the first antibody bound to the surface. This avoids binding of the antibody to more than one antigen. Additionally, the present invention includes a second antibody which binds to the first antibody-analyte complex. This improves the sensitivity with which the analytes are detected in the sample.

According to an embodiment, the nucleic acid fragment comprising the exposed 3' hydroxyl group and the nucleic acid fragment comprising the 5' exposed phosphate group are in close proximity to each other. Therefore, ligation of the two nucleic acid fragments is enabled due to proximity.

According to another embodiment, ligating the nucleic acid fragments includes introducing a linker nucleic acid fragment to the first antibody-analyte-second antibody complex. The linker nucleic acid fragment may be complementary to the nucleic acid fragment comprising the exposed 3' hydroxyl group and the nucleic acid fragment comprising the 5' exposed phosphate group. A ligase enzyme may be added to the linker nucleic acid and the first antibody-analyte-second antibody complex to form a mixture. The ligase enzyme catalyzes the ligation of the two nucleic acid fragments and the linker nucleic acid fragment. The mixture is further incubated so as to enable the linker nucleic acid fragment to connect the nucleic acid fragment comprising the exposed 3' hydroxyl group and the nucleic acid fragment comprising the 5' exposed phosphate group.

According to yet another embodiment, amplifying the ligated nucleic acid fragments includes performing an amplification that is quantitative and is not limited to but includes polymerase chain reaction or isothermal amplification on the ligated nucleic acid fragments.

According to a further embodiment, the method further includes pre-amplifying the ligated nucleic acid fragments before performing a quantitative amplification. Pre-amplification of the ligated nucleic acid fragments enables an increase in the sensitivity of the quantitative polymerase chain reaction or any other amplification method.

According to another embodiment, the surface to which the first antibody is bound is a spherical bead. Therefore, the spherical bead may be uniformly coated with the antibodies.

According to yet another embodiment, the sample may be chosen from a group including, but not limited to, whole blood, sputum, urine, cerebrospinal fluid and bronchoalveolar lavage.

In another aspect, the invention relates to a kit for detecting one or more analytes in a sample. The kit includes a surface bound to one or more first antibodies, wherein the surface is bound to at least one portion of each first antibody, a second antibody conjugated to a nucleic acid fragment comprising an exposed 3' hydroxyl group, and another second antibody conjugated to a nucleic acid fragment comprising an exposed 5' phosphate group.

According to an embodiment, the kit further includes one or more enzymes for ligating the nucleic acid fragments conjugated to the secondary antibody.

According to an embodiment, the kit may further include one or more linker nucleic acid fragments that may be complementary to the nucleic acid fragment comprising the exposed 3' hydroxyl group and the nucleic acid fragment comprising the exposed 5' phosphate group.

### BRIEF DESCRIPTION OF THE DRAWINGS

The present invention is further described hereinafter with reference to illustrated embodiments shown in the accompanying drawings, in which:
Figure 1 illustrates a flowchart of a method of detecting one or more analytes in a sample, according to an embodiment of the invention.
Figure 2A illustrates a schematic diagram of a method of detecting one or more analytes in a sample, according to an embodiment of the invention.
Figure 2B illustrates a schematic diagram of a method of detecting one or more analytes in a sample, according to another embodiment of the invention.
Figure 3 illustrates a flowchart of a method of ligating the nucleic acid fragments, according to an embodiment.

### DETAILED DESCRIPTION

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

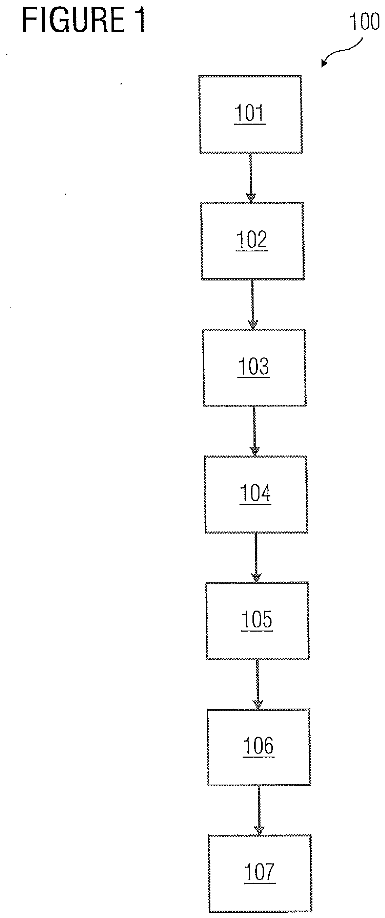

Figure 1 illustrates a flowchart of a method 100 of detecting one or more analytes in a given sample. The analytes, may be, for example, any antigen for which one or more antibodies can be produced. The analytes may include, for example, pathogens such as bacteria, viruses, etc., protein molecules associated with pathogens, autoantigens generated by an individual as a result of an autoimmune disorder, protein biomarkers for any specific disease present in physiological fluids etc. The sample may be any physiological fluid such as whole blood, urine, sputum, bronchoalveolar lavage, cerebrospinal fluid, etc. that may be capable of carrying the one or more analytes. At step 101 of the method 100, the sample comprising the one or more analytes is introduced to a surface bound to at least one portion of a first antibody. The surface may possess properties such that multiple bound first antibodies are in close proximity to each other. The surface may be, for example, spherical in shape thereby allowing the at least one portion of multiple first antibodies to be in close proximity to each other. The at least one portion of the first antibody binds with the one or more analytes present in the sample to form a first antibody-analyte complex. The first antibody is chosen such that the first antibody has an affinity to the one or more analytes present in the sample. The first antibody may also include, for example, aptamers, nanobodies, or any other derivatives of antibodies. In an embodiment, the surface may be bound to the one or more first antibody as a whole component. In another embodiment, the surface may be bound to the first antibody such that an antigen binding fragment (Fab) of the first antibody is exposed. At step 102 of the method 100, the first antibody-analyte complex is incubated with second antibodies. The concentration of the second antibodies may be stoichiometrically determined. Each second antibody may be conjugated with one or more nucleic acid fragment. The second antibody may be chosen such that the second antibody has an affinity for the one or more analytes present in the sample. The at least one portion of each second antibody binds to the first antibody-analyte complex to form an agglutination complex or a first antibody-analyte-second antibody complex such that the analyte is sandwiched between the first antibody and the second antibody. The one or more nucleic acid fragments conjugated to each second antibody may have at least one of an exposed 3' hydroxyl group or an exposed 5' phosphate group. In an embodiment, the properties of the surface to which the at least one portion of the first antibody is bound further enables increased proximity between the nucleic acid fragments conjugated to the at least one portion of the second antibody.

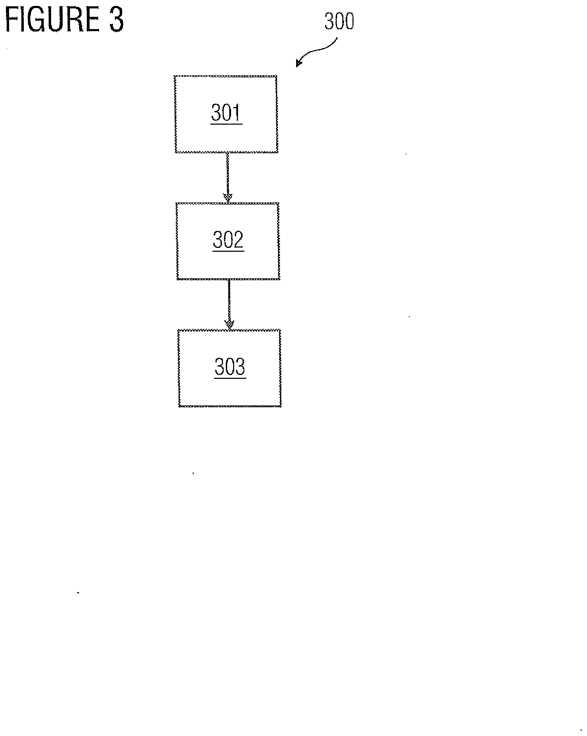

At step 103 of the method 100, the one or more nucleic acid fragments conjugated to the at least one portion of the second antibody are ligated. The method steps related to ligation of the one or more nucleic acid fragments is described in detail in Figure 3. Referring to Figure 3, at step 301 of method 300, one or more linker nucleic acid fragments is introduced to the first antibody-analyte-second antibody complex. The linker nucleic acid fragment may be a short nucleic acid fragment composed of 10-15 nucleotides. In an embodiment, the linker nucleic acid fragment may be chosen such that the linker nucleic acid fragment is complementary to the one or more nucleic acid fragments conjugated to the at least one portion of the second antibody. In a further embodiment, the linker nucleic acid fragment may be partly complementary to the nucleic acid fragment including the exposed 3' hydroxyl group and partly complementary to the nucleic acid fragment including the exposed 5' phosphate group. At step 302, ligase enzyme and the linker nucleic acid fragment is added to the first antibody-analyte-second antibody complex to form a mixture. Ligase enzyme acts as a catalyst in ligation of the nucleic acid fragments conjugated to the at least one portion of the second antibody to the linker nucleic acid fragment. At step 403, the mixture is incubated such that the linker nucleic acid fragment binds the nucleic acid fragment comprising the exposed 3' hydroxyl group and the nucleic acid fragment comprising the 5' exposed phosphate group. In an embodiment, the mixture may be incubated for about one minute at 50-60°C to enable hybridization of linker DNA to the oligos, also thereby bringing the exposed ends of the nucleic acid fragments proximal to each other. Advantageously, the properties associated with the surface to which the at least one portion of the first antibody is bound provides for effective ligation of the nucleic acid fragments conjugated to the second antibody due to the proximity of nucleic acid induced by the surface bound first antibody-analyte-second antibody complex. Due to the surface properties, the nucleic acid fragments bound to the second antibody are in close proximity to each other, thereby enabling effective ligation.

At step 104 of the method 100, the ligated nucleic acid fragments are separated from the first antibody-analyte-second antibody complex. In an embodiment, the one or more nucleic acid fragments may be conjugated to the second antibody through a bridging oligonucleotide. Therefore, the bridging oligonucleotide may be broken down to separate the ligated nucleic acid fragments from the first antibody-analyte-second antibody complex. Alternatively, the separation of the ligated nucleic acid fragments may also be achieved during the process of amplification. At step 105, the nucleic acid fragments are pre-amplified using polymerase chain reaction. The polymerase chain reaction based pre-amplification of the separated nucleic acid fragments enables increase in the sensitivity of the detection of the analytes in the sample. The process of pre-amplification of nucleic acid fragments using polymerase chain reaction is well known in the art and is therefore not elaborated upon in the description. In an alternate embodiment, the nucleic acid fragments may be amplified using any other amplification method. At step 106, the ligated nucleic acid fragments are amplified. This amplification may be performed using, but not limited to, for example, real-time polymerase chain reaction. Real-time polymerase chain reaction or quantitative polymerase chain reaction enables real-time determination of quantity of the amplified nucleic acid. In an embodiment, real-time polymerase chain reaction may use a fluorescent dye labelled probe during the process of amplification of the nucleic acid fragments. As the number of copies of the amplified nucleic acid fragments increase, intensity of fluorescence generated also increases. At step 107, the one or more analytes present in the sample is detected. The detection may be based on, for example, the amount of fluorescence generated through real-time polymerase chain reaction.

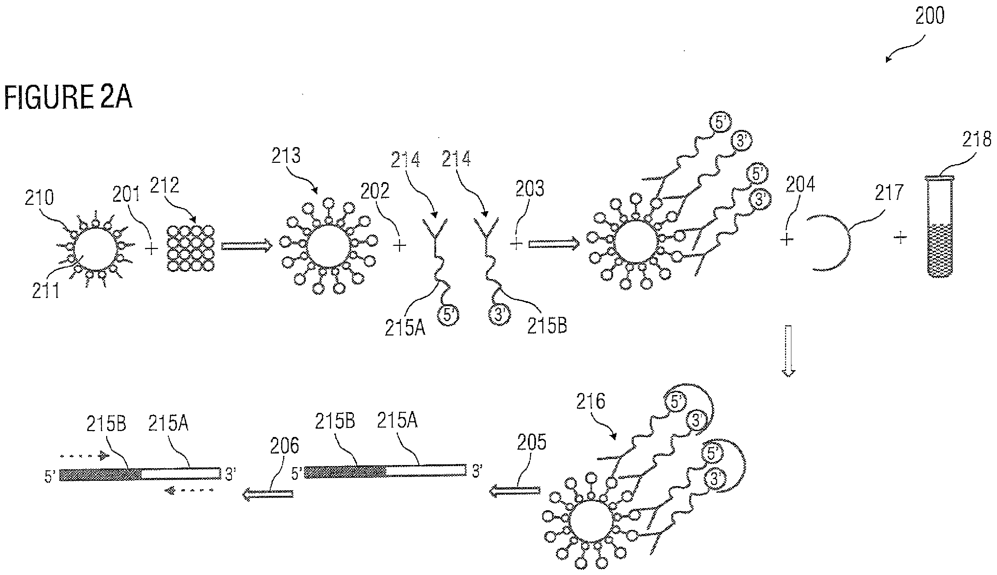

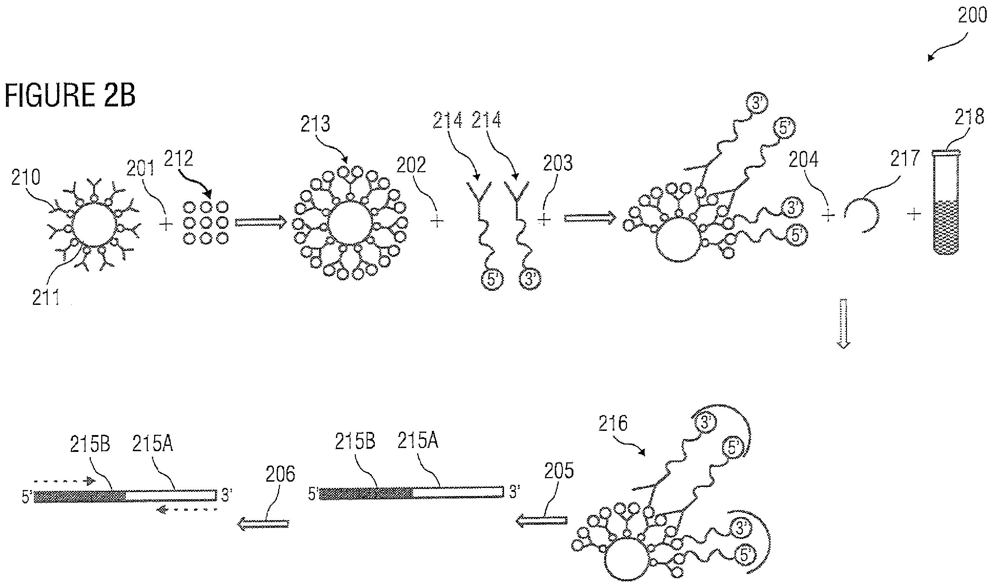

Figure 2A and 2B illustrate schematic diagrams of the method 200 of detecting one or more analytes in a sample, according to two embodiments. Figure 2A depicts a first antibody 210 as an analyte-specific Fab molecule conjugated to the surface 211 of a bead or dendrimer-like structure, while Figure 2B depicts a first antibody 210 as an analyte-specific antibody molecule conjugated to the surface 211 of a bead or dendrimer-like structure. One portion of a first antibody 210 is bound to a surface 211 such that another portion of the first antibody 210 is exposed to bind to one or more analytes 212 in the sample. In Figure 2A, the exposed portion of the first antibody 210 is an antigen binding fragment (Fab) region of the first antibody. The surface 211 may be spherical in shape and may be composed of polystyrene, silica, glass, etc. The spherical shape of the surface 211 enables effective agglutination of the at least one portion of the first antibody 210 with the one or more analytes 212 in the sample. The surface 211 may also include, for example, antibody dendrimers. The dendrimeric surface 211 has a spherical core and a dendritic structure or branches originating from the spherical core. Advantageously, the dendrimeric surface is capable of binding to a plurality of first antibodies. Therefore, antibody exposure to analytes present in the sample is increased. In an alternate embodiment, as illustrated in Figure 2B, the first antibody 210 may be bound to the surface 211 as a whole component. At step 201, the analytes 212 bind to the first antibody 210 when exposed to the surface 211 bound with at least one portion of the first antibody 210. A first antibody-analyte complex 213 is formed. At step 202, a set of second antibodies 214 is introduced to the first antibody-analyte complex 213, where the set of second antibodies is conjugated with two half DNA oligos: one half-oligo has a 5' phosphate exposed group (215A), while the second half has a 3' hydroxyl exposed group (215B). The second antibodies 214 are chosen such that each second antibody has an affinity for the one or more analytes 212. Each second antibody 214 is respectively conjugated to one or more nucleic acid fragments 215A, 215B. The nucleic acid fragments may include at least one of an exposed 3' hydroxyl group or an exposed 5' phosphate group. On incubation, the second antibodies 214 along with the one or nucleic acid fragments 215A, 215B bind to the first antibody-analyte complex 213 to generate first antibody-analyte-second antibody complex 216. At step 203, one or more linker nucleic acid fragments 217 and ligase enzyme 218 are added to the first antibody-analyte-second antibody complex 216. The linker nucleic acid fragments 217 is chosen such that each nucleic acid fragments 215A, 215B are complementary to the linker nucleic acid fragments 217, preferably 10-15 base pairs in length. The ligase enzyme 218 acts as a catalyst in the ligation process, with an incubation time of around 5-15 minutes from ligase enzyme addition, thereby resulting in ligating the nucleic acid fragments 215A, 215B conjugated to the second antibody 214 with the linker nucleic acid fragment 217 acting as a connecting agent. Advantageously, due to the spherical or dendrimeric format of the surface 211, the nucleic acid fragments 215A, 215B conjugated to the second antibodies 214 come in close proximity to each other in the first antibody-analyte-second antibody complex 216. Therefore, the ligation process is made more effective due to proximity. At step 204, the ligated nucleic acid fragments 215A, 215B are separated from the first antibody-analyte-second antibody complex 216, for example, through selective hydrolysis, and subjected to pre-amplification using polymerase chain reaction. The pre-amplification step improves the sensitivity of detection of analytes 212 present in the sample. At step 205, the pre-amplified nucleic acid fragments 215A, 215B are amplified using real-time polymerase chain reaction or any other isothermal amplification method. At step 206, the fluorescence generated during the real-time polymerase chain reaction is used to detect and quantity the one or more analytes 212 present in the sample.

The advantage of the invention is that sensitivity of detection of the one or more analytes in the sample in improved. Therefore, the invention enables detection of analytes in the sample to the level of picogram/µL and femtogram/µL. Additionally, the invention eliminates the need for multiple wash steps to remove background nucleic acid information. Therefore, the method steps can be carried out using a single equipment without intervening wash steps. Furthermore, the invention is compatible with molecular test platform. Therefore, nucleic acids and proteins may be detected using a single platform. This enables ease of workflow and allows for single sample collection for detection of nucleic acids and proteins.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein; rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

## Claims

1. Use of a kit to carry out a method of detecting one or more analytes in a sample,
wherein the kit comprising:
a surface (211) bound to one or more first antibodies, wherein the surface is bound to at least one portion of each first antibody;
a second antibody (214) conjugated with a nucleic acid fragment comprising an exposed 3' hydroxyl group; and
another second antibody (214) conjugated with a nucleic acid fragment comprising an exposed 5' phosphate group, and
an alkaline agent for hydrolyzing at least one oligonucleotide which binds the nucleic acid fragment comprising the exposed 3' hydroxyl group (215B) and the nucleic acid fragment comprising the 5' exposed phosphate group (215A) to the at least one portion of the second antibody (214) thereby separating the ligated nucleic acid fragments, and
wherein the method comprises
introducing the sample to a surface (211) bound to one or more first antibodies (210), wherein the surface (211) is bound to at least one portion of each first antibody to form a first antibody-analyte complex (213);
forming a first antibody-analyte-second antibody complex (216) by incubating the first antibody-analyte complex (213) with (a) at least one portion of a second antibody (214) conjugated with a nucleic acid fragment comprising an exposed 3' hydroxyl group (215B) and (b) at least one portion of another second antibody conjugated with a nucleic acid fragment comprising an exposed 5' phosphate group (215A);
ligating the nucleic acid fragment comprising the exposed 3' hydroxyl group (215B) and the nucleic acid fragment comprising the exposed 5' phosphate group (215A);
separating the ligated nucleic acid fragments (215A, 215B) from the first antibody-analyte-second antibody complex (216);
amplifying the separated ligated nucleic acid fragments (215A, 215B); and
detecting the one or more analytes present in the sample based on the amplified nucleic acid fragments, wherein the nucleic acid fragment comprising the exposed 3' hydroxyl group (215B) and the nucleic acid fragment comprising the exposed 5' phosphate group (215A) are in close proximity to each other.

2. The use according to claim 1, wherein the kit further comprising one or more enzymes for ligating the nucleic acid fragment comprising the exposed 3' hydroxyl group (215B) and the nucleic acid fragment comprising the exposed 5' phosphate group (215A).

3. The use according to claim 1, wherein the kit further comprising one or more linker nucleic acid fragments (217) complementary to the nucleic acid fragment comprising the exposed 3' hydroxyl group (215B) and the nucleic acid fragment comprising the exposed 5' phosphate group (215A).

4. The use according to claim 1, wherein the nucleic acid fragment comprising the exposed 3' hydroxyl group (215B) and the nucleic acid fragment comprising the exposed 5' phosphate group (215A) are ligated through enzymatic or chemical process.

5. The use according to claim 4, wherein ligating the nucleic acid fragment comprising the exposed 3' hydroxyl group (215B) and the nucleic acid fragment comprising the 5' exposed phosphate group (215A) through enzymatic process further comprises:
introducing one or more linker nucleic acid fragment (217) to the first antibody-analyte-second antibody complex (216);
adding ligase enzyme (218) to the linker nucleic acid fragment (217) and the first antibody-analyte-second antibody complex (216) to form a mixture; and
incubating the mixture to ligate the nucleic acid fragment comprising the exposed 3' hydroxyl group (215B) and the nucleic acid fragment comprising the 5' exposed phosphate group (215A), wherein the linker nucleic acid fragment (217) connects the nucleic acid fragment comprising the exposed 3' hydroxyl group (215B) and the nucleic acid fragment comprising the 5' exposed phosphate group (215A).

6. The use according to claim 1, wherein amplifying the ligated nucleic acid fragments (215A, 215B) comprises performing a quantitative amplification on the ligated nucleic acid fragments.

7. The use according to claim 6, further comprising pre-amplifying the ligated nucleic acid fragments (215A, 215B) before performing the quantitative amplification.

8. The use according to claim 1, wherein the surface (211) to which the one or more first antibodies are bound is at least one of spherical or dendrimer in format.

9. The Use according to claim 1, wherein the sample is chosen from a group comprising but not limited to whole blood, sputum, urine, cerebrospinal fluid, and bronchoalveolar lavage.
